# FASCICULE DE BREVET EUROPEEN

(11) **EP 0 527 081 B1**
(45) Date de publication et mention de la délivrance du brevet: **03.01.1996**
(21) Numéro de dépôt: 92402218.9
(22) Date de dépôt: 03.08.1992
(51) Int. Cl.: C07D 265/22, C07D 413/12, A61K 31/535

(54) **Nouvelles pipérazinylalcoyl-3 dihydro-2,3 4H-benzoxazine-1,3 ones-4 substituées, leur préparation et leur application en thérapeutique**
Substituierte 3-Piperazinylalkyl 2,3-Dihydro-4H-1,3-Benzoxazin-4-One, deren Herstellung und Verwendung in Heilkunde
Substituted 3-piperazinylalkyl-2,3-dihydro-4H-1,3-benzoxazine-4-ones, their preparation and their therapeutical use

(30) Priorité: 05.08.1991 FR 9109946
(43) Date de publication de la demande: 10.02.1993
(73) Titulaire: PIERRE FABRE MEDICAMENT, F-92100 Boulogne (FR)
(72) Inventeur: Rieu, Jean-Pierre, F-81100 Castres (FR); Bigg, Dennis, F-81100 Castres (FR)
(74) Mandataire: Ahner, Francis

(56) Documents cités:
- EP-A- 0 233 728
- EP-A- 0 359 627

## Description

La présente invention réalisée au Centre de Recherche PIERRE FABRE a pour objet de nouvelles pipérazinylalcoyl-3 dihydro-2,3 4H-benzoxazine-1,3 ones-4 substituées, leur procédé de préparation et leur utilisation en tant que médicaments utiles principalement dans les troubles cardiovasculaires et du système nerveux.

Dans une étude précédente, nous avons montré que des dérivés des arylalcoylaminoalcoyl-3 4H-benzoxazine-1,3 ones-4 possédent d'excellentes propriétés en thérapeutique cardiovasculaire et notamment dans le traitement des tachycardies, du trouble du rythme cardiaque et de l'ischémie (brevet EP 359627). Lors des modulations structurales au niveau de la fonction amine, nous avons remarqué que la substitution d'une phénylalcoylamine par une arylpipérazine (composés de la présente invention) conduisait à des dérivés qui avaient pratiquement perdu leur activité bradycardisante mais qui présentaient en contre partie une puissante affinité pour les récepteurs 5HT et plus précisément pour les 5HT₁A.

Les développements récents des concepts sur l'étude de la 5HT comme neuromédiateur et de ses récepteurs ont conduit à une recherche très dynamique sur des composés possédant une affinité potentielle pour ces récepteurs se traduisant par une action soit au niveau cardiaque, soit sur le système nerveux central. La découverte d'un grand nombre de composés a justifié la publication de mises au point récentes sur leurs propriétés pharmacologiques et le rôle de la sérotonine dans le mode d'action (cf. R.A. Glennon : Central Serotonin Receptors as Targets for Drug Research, J. Med. Chem. 30 (1) 1-12 (1987) ; P.R. Saxena and C.M. Villalon : Cardiovascular Effects of Serotonin Agonists and Antagonists, J. Cardiov. Pharmacol. 16 (suppl.7) S17-S34 (1990) ; I. Van Vijngaarden, M.Th.M. Tulp and W. Soudijn : The Concept of Selectivity in 5HT Receptor Research, Eur. J. Pharmacol. - Molecular Pharmacol. Section 188, 301-312 (1990) ; S.J. Peroutka : Receptor Families for 5HT, J. Cardiov. Pharmacol. 16 (suppl.3) S8-S14 (1990) ; J.R. Fozard : 5HT, The Enigma variations, T.I.P.S. 8 (12) 501-506 (1987) etc...).

Contrairement aux imides cycliques des composés précédemment décrits et renfermant un groupement pipérazinylalcoyle (cf. H. Wilkström and K. Svensson, Annual Reports in Medicinal Chemistry vol 25, 41-50, Academic Press Inc (1989), les composés de la présente invention qui sont des dérivés de pipérazinylalcoyl-3 4H-Benzoxazine-1,3 ones-4 de structure orignale possèdent une seule fonction carbonyle dans le cycle et une puissante affinité pour les récepteurs de la 5-hydroxytryptamine dont la sélectivité est fonction des substituants. Ainsi ces composés peuvent être utilisés dans les troubles du système nerveux central comme anxiolytiques, antidépresseurs ou antimigraineux ainsi qu'en thérapeutique cardiovasculaire comme antihypertenseurs.

### MOLECULES REVENDIQUEES

La présente invention a plus particulièrement pour objet les pipérazinylalcoyl-3 4H-benzoxazine-1,3 ones-4 de structure I 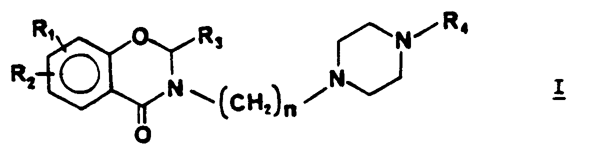
dans laquelle :
- R₁ et R₂ égaux ou différents représentent un hydrogène, un halogène, un groupement alcoyle renfermant de 1 à 6C, un groupement alcoyloxy renfermant de 1 à 4C, un hydroxyle, un groupement nitro, un amino substitué ou non par un groupement acyle ou un ou deux alcoyle renfermant chacun indépendamment 1 à 4C ; un groupement methylsulfonylamino ;
- R₃ représente un hydrogène ou un groupement aliphatique ramifié ou non, saturé ou non, renfermant 1 à 5 C ;
- R₄ représente soit :
   . un groupement phényle substitué ou non par un ou plusieurs radicaux identiques ou différents, choisis parmi un atome d'halogène, un groupement alcoyle contenant de 1 à 6 C ; un groupement alcoyloxy renfermant de 1 à 6 C ; un groupement hydroxy, un trifluorométhyle, un groupement nitro ou un radical amino substitué ou non par un ou plusieurs groupements alcoyle légers, acyle ou carboxylate, renfermant tous trois de 1 à 4 atomes de carbone, un groupement méthylsulfonylamino;
   . un groupenent hétéroaryle monocyclique renfermant un à deux atomes d'azote ;
- n peut prendre les valeurs de 2 à 6.

La présente invention inclut aussi les sels minéraux ou organiques thérapeutiquement acceptables des composés de formule I, éventuellement sous forme hydratée. Lorsque les composés de formule générale I renfermant un carbone asymétrique, la présente invention concerne aussi bien les racémates que les différents énantiomères, ou leurs mélanges. Les radicaux R₁ et R₂ sont préférentiellement choisis parmi les suivants : H, CH₃, CH₃CH₂, (CH₃)₂CH, CH₃O, CH₃CH₂O, Br, Cl, F, OH, NO₂, NH₂, CH₃CONH, CH₃SO₂NH, (CH₃)₂N. Le radical R₃ est de façon préférentielle représenté par : H, CH₃, CH₃CH₂, (CH₃)₂CH, CH₃-CH=CH.
Le radical R₄ est plus particulièrement représenté par un groupement choisi parmi :
- un phényle pouvant être ou non substitué par un ou plusieurs radicaux suivants : Br, Cl, F, CF₃, CH₃, CH₃CH₂, CH₃O, CH₃CH₂O, NO₂, OH, NH₂, CH₃CONH, CH₃SO₂NH, (CH₃)₂N,
- un groupement pyridyle,
- un groupement pyrimidinyle.

La présente invention concerne également l'utilisation des composés de formule générale I à titre de médicament et les compositions pharmaceutiques les renfermant. Les compositions pharmaceutiques selon la présente invention peuvent utiliser un ou plusieurs composés de formule I éventuellement en association avec un ou plusieurs autres principes actifs. Enfin les procédés de synthèse des composés de formule générale I font aussi partie de la présente invention.

### PROCEDE DE PREPARATION DES COMPOSES DE FORMULE I

La meilleurs méthode de préparation des dérivés de formule générale I consiste à condenser un composé de formule II convenablement substitué avec une pipérazine de formule III selon le schéma : 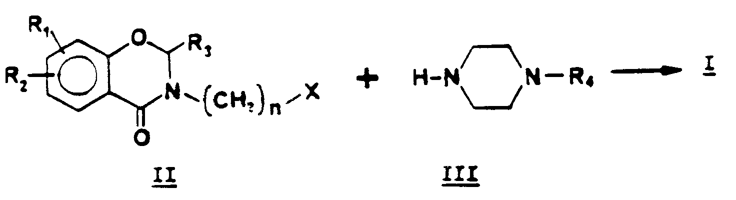
où R₁ à R₄ et n ont la même signification que dans la formule générale I et où X représente un halogène (Cl, Br, I) ou bien un groupement alcanesulfonate, préférentiellement le mésylate (MeSO₃) ou un groupement arylsulfonate, notamment le tosylate (p-MeC₆H₄SO₃). La condensation est réalisée de préférence à une température comprise entre 20 et 110°C en utilisant ou non un excès d'amine III et en présence ou non d'une base, choisie de façon préférentielle parmi la pyridine ou une amine tertiaire comme, par exemple, la triéthylamine.

Les produits de la réaction précédente peuvent être salifiées avec l'acide souhaité dans un solvant apolaire ou polaire comme l'acétate d'éthyle ou l'éthanol à titre d'exemple pour donner les mono sels ou di sels attendus et leurs hydrates éventuels.

Les exemples suivants illustrent l'invention sans toutefois en limiter la portée.

### Exemple 1

### Monochlorhydrate de dihydro-2,3 [((trifluorométhyl-3 phényl)-4 pipérazinyl)-4 butyl]-3 4H-Benzoxazine-1,3 one-4

Un mélange de 2,84 g (9,4 mmoles) de dihydro-2,3 (méthanesulfonyloxy-4 butyl)-3 4H-Benzoxazine-1,3 one-4 et de 3,27 g (14,2 mmoles) de (trifluorométhyl-3 phényl)-4 pipérazine dans 1,32 ml (0,96 g ou 9,4 mmoles) de triéthylamine est chauffé à 60°C pendant 1 h 15. Après retour à 25°, le mélange est versé dans 80 ml d'eau et le pH est amené à 4,5 par addition d'acide chlorhydrique concentré. Le dérivé attendu est extrait à l'acétate d'éthyle 2 X 50 ml et la solution organique est lavée avec de l'eau acidulée à pH 4,5 puis à l'eau salée saturée et séchée sur sulfate de sodium. Après évaporation à siccité, le résidu (m = 3,55 g) est repris dans l'acétate d'éthyle, refroidi sur bain de glace puis traité jusqu'à pH 2 avec une solution d'acide chlorhydrique dans l'acétate d'éthyle. Les cristaux formés sont filtrés, essorés et rincés à l'éther isopropylique. m = 2,76 g (Rdt = 72 %) de poudre blanche de formule 1 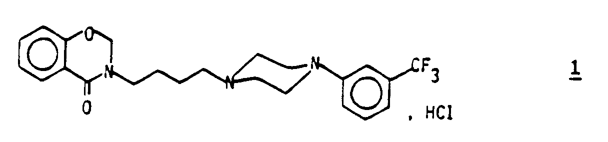
- formule brute : C₂₃H₂₇ClF₃N₃O₂
- masse moléculaire : 469,935
- cristaux blanc pulvérulents
- point de fusion : 156°C
- IR (KBr) √CO amide : 1675 cm⁻¹
- RMN (CDCl₃) δ : 1.69-2.15 (m,4H) ; 2.85-3.2 (m,4H) ; 3.55-3.90 (m,8H) ; 5.21 (s,2H) ; 6.85-7.26 (m,5H) ; 7.30-7.6 (m,2H) ; 7.92 (dd,1H) ; 12.76 (s,1H) ; p.p.m.
- soluble à 6 % dans le DMSO.

### Exemple 2

### Monochlorhydrate de dihydro-2,3 [(phényl-4 pipérazinyl)-3 propyl]-3 4H-Benzoxazine-1,3 one-4

Un mélange de 4,91 g (17,2 mmoles) de dihydro-2,3 (méthanesulfonyloxy-3 propyl)-3 4H-Benzoxazine-1,3 one-4 et de 8,37 g (51,6 mmoles) de phényl)-4 pipérazine contenu dans un évaporateur rotatif à boules est chauffé à 50°C pendant 30 mn puis l'amine est distillée à 50°C sous 10⁻³m.bar. Le résidu solide est repris dans 40 ml d'eau et 50 ml de toluène et le pH est amené à 4 par addition de HCl, les phases sont séparées et l'extraction par le toluène à pH 4 est renouvelée 3 fois. Les phases organiques sont réunies puis la phase totale est lavée à l'eau salée, séchée sur sulfate de sodium et évaporée à siccité. La base résiduelle (m = 4,8 g) est reprise dans un mélange de 40 ml AcOEt et 10 ml d'alcool isopropylique, après refroidissement à 0°, on ajoute une solution d'acide chlorhydrique dans l'acétate d'éthyle jusqu'à pH 2. Le précipité formé est filtré, essoré, séché à l'étuve à 50° (m=4,5 g). Les cristaux sont recristallisés 2 fois d'un mélange AcOEt/iPrOH 40/60 pour donner 3,6 g (Rdt = 54 %) de cristaux blancs de formule **2** 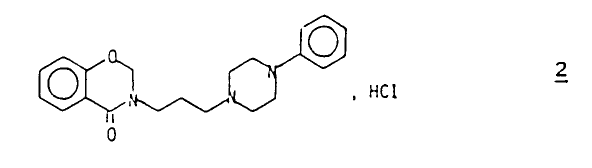
- formule brute : C₂₁H₂₆ClN₃O₂
- masse moléculaire : 387,911
- cristaux blanc cassé
- point de fusion : 190°C
- IR (KBr) √CO amide : 1670 cm⁻¹
- RMN (CDCl₃) δ : 2.20-2.43 (m,2H) ; 2.9-3.2 (m,4H) ; 3.4-3.8 (m,8H) ; 5.28 (s,2H) ; 6.8-7.50 (m,7H) ; 7.88 (d,1H) ; 12.67 (s,1H) ; p.p.m.
- soluble à 1 % dans l'eau.

### Exemple 3

### Dichlorhydrate de dihydro-2,3 [(phényl)-4 pipérazinyl)-2 éthyl]-3 4H-Benzoxazine-1,3 one-4

En utilisant le mode opératoire décrit dans l'exemple 2 mais en partant de la dihydro-2,3 (méthanesulfonyloxy-2 éthyl)-3 4H-Benzoxazine-1,3 one-4, on obtient avec un rendement de 48 % le composé **3** de formule : 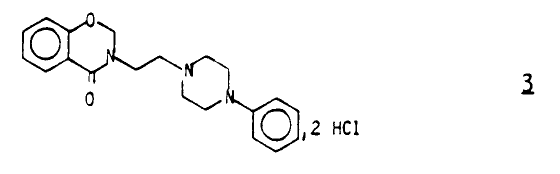
- formule brute : C₂₀H₂₅Cl₂N₃O₂
- masse moléculaire : 410,345
- cristaux blancs
- point de fusion : 206-208°C
- IR (KBr) √CO amide : 1666 cm⁻¹
- RMN (DMSO d₆) δ : 3.1-3.50 (m,6H) ; 3.6-4.0 (m,6H) ; 5.47 (s,2H) ; 6.88 (t,1H) ; 6.95-7.4 (m,6H) ; 7.54 (t,1H) ; 7.81 (d,1H) ; 11.48 (s,1H) ; 12.32 (s,1H) p.p.m.
- soluble dans l'eau à 1 %.

### Exemple 4

### Chlorhydrate de dihydro-2,3 [(phényl-4 pipérazinyl)-4 butyl]-3 4H-Benzoxazine-1,3 one-4

Ce composé est préparé selon le procédé décrit dans l'exemple 2 mais à partir de la dihydro-2,3 (méthanesulfonyloxy-4 butyl)-3 4H-Benzoxazine-1,3 one-4 avec un rendement de 65 % et de formule **4** : 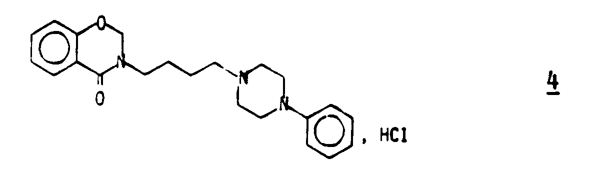
- formule brute : C₂₂H₂₈ClN₃O₂
- masse moléculaire : 401,938
- cristaux blancs
- point de fusion : 218-220°C
- IR (KBr) √CO amide : 1670 cm⁻¹
- RMN (CDCl₃) δ : 1.45-1.67 (m,2H) ; 1.68-1.90 (m,2H) ; 2.7-3.5 (m,12H) ; 5.02 (s,2H) ; 6.6-6.8 (m,4H) ; 6.9 (t,1H) ; 7.07 (t,2H) ; 7.15-7.28 (m,1H) ; 7.68 (dd,1H); 12.14 (s,1H) p.p.m.
- soluble dans l'eau à 0,5 %.

### Exemple 5

### Dichlorhydrate de dihydro-2,3 [(méthoxy-2 phényl)-4 pipérazinyl)-2 éthyl]-3 4H-Benzoxazine-1,3 one-4

Selon le procédé décrit dans l'exemple 1 mais en partant de (méthoxy-2 phényl)-1 pipérazine et de la dihydro-2,3 (méthanesulfonyloxy-2 éthyl)-3 4H-Benzoxazine-1,3 one-4, on obtient après recristallisation de l'alcool isopropylique le composé **5** avec un rendement de 58 % et de formule : 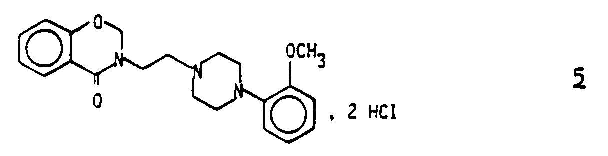
- formule brute : C₂₁H₂₇Cl₂N₃O₃
- masse moléculaire : 440,451
- cristaux blancs
- point de fusion : 206°C
- IR (KBr) √CO amide : 1655 cm⁻¹
- RMN (base) (CDCl₃) δ : 2.5-2.8 (m,6H) ; 2.9-3.2 (m,4H); 3.75 (t,2H) ; 3.86 (s,3H) ; 5.31 (s,2H) ; 6.7-7.20 (m,6H) ; 7.3-7.6 (m,1H) ; 7.97 (dd,1H) ; p.p.m.
- soluble dans l'eau à 6 %.

Le monochlorhydrate a aussi été isolé.
- formule brute : C₂₁H₂₆ClN₃O₃
- masse moléculaire : 403,91
- cristaux blancs
- point de décomposition lente : 206-207°C
- soluble dans l'eau à 0,9 %.

### Exemple 6

### Chlorhydrate de dihydro-2,3 [((méthoxy-2 phényl)-4 pipérazinyl)-3 propyl]-3 4H-Benzoxazine-1,3 one-4

L'application du procédé décrit dans l'exemple 1 à la (méthoxy-2 phényl) pipérazine conduit, avec un rendement de 42 % au composé **6** ayant pour formule : 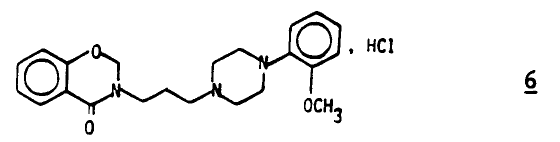
- formule brute : C₂₂H₂₈ClN₃O₃
- masse moléculaire : 417,937
- poudre blanche
- point de fusion lent : 190°C
- IR (KBr) √CO amide : 1660 cm⁻¹
- RMN (CDCl₃) δ : 2.2-2.5 (m,2H) ; 2.8-3.5 (m,4H) ; 3.6-4 (m,11H) ; 5.3 (s,2H) ; 6.8-7.2 (m,6H) ; 7.3-7.6 (m,1H) ; 7.9 (dd,1H) ; 12.6 (s,1H) ; p.p.m.
- soluble dans l'eau à 0,5 %.

### Exemple 7

### Monohydrate du dichlorhydrate de dihydro-2,3 [(méthoxy-2 phényl)-4 pipérazinyl)-4 butyl]-3 4H-Benzoxazine-1,3 one-4

En adaptant le procédé de l'exemple 1 à la (méthoxy-2 phényl) pipérazine et à la dihydro-2,3 (méthanesulfonyloxy-4 butyl)-3 4H-Benzoxazine-1,3 one-4, on obtient après recristallisation de l'alcool isopropylique, avec un rendement de 61 %, le composé **7** de formule : 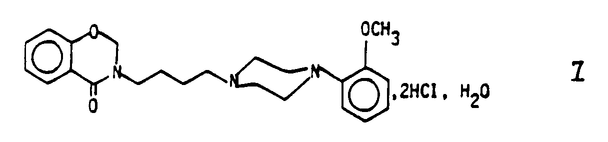
- formule brute : C₂₃H₃₃Cl₂N₃O₄
- masse moléculaire : 486,44
- cristaux blanc cassé
- point de fusion : 202°C
- IR (KBr) √CO amide : 1665 cm⁻¹
- RMN (CDCl₃) δ : 1.5-2.1 (m,4H) ; 2-3.2 (m,2H) ; 3.2-4.6 (m,6H) ; 3.95 (s,3H) ; 4-4.3 (m,2H) ; 4.85 (t,2H) ; 5.14 (s,2H) ; 6.2-7.6 (m,6H + H₂O) ; 7.81 (dd,1H) ; 8,00 (dd,1H) ; 13.12 (s,1H) ; p.p.m.
- soluble dans l'eau à 3 %.

### Exemple 8

### Monochlorhydrate de dihydro-2,3 [(méthyl-2 phényl)-4 pipérazinyl)-2 éthyl]-3 4H-Benzoxazine-1,3 one-4

En partant de la (méthyl-2 phényl) pipérazine et de la dihydro-2,3 (méthanesulfonyloxy-2 éthyl)-3 4H-Benzoxazine-1,3 one-4, et en opérant selon le mode opératoire décrit dans l'exemple 1, on obtient apis recristallisation du méthanol, avec un rendement de 71 %, le composé **8** ayant pour formule : 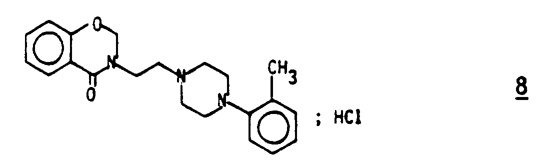
- formule brute : C₂₁H₂₆ClN₃O₂
- masse moléculaire : 387,89
- cristaux prismatiques blancs
- point de fusion : 216°C
- IR (KBr) √CO amide : 1661 cm⁻¹
- RMN (CDCl₃) δ : 2.27 (s,3H) ; 2.9-3.2 (m,4H) ; 3.25-3.85 (m,6H) ; 4.21 (t,3H) ; 5.45 (s,2H) ; 6.80-7.3 (m,6H) ; 7.35-8.6 (m,1H) ; 7.91 (dd,1H) ; 12.9 (s,1H) ; p.p.m.
- soluble dans l'eau à 0,35 %.

### Exemple 9

### Chlorhydrate de dihydro-2,3 [(chloro-3 phényl)-4 pipérazinyl)-2 éthyl]-3 4H-Benzoxazine-1,3 one-4

L'application du mode opératoire décrit dans l'exemple 1 à la chloro-3 phénylpipérazine et à la dihydro-2,3 (méthanesulfonyloxy-2 éthyl)-3 4H-Benzoxazine-1,3 one-4, permet de préparer, après purification dans le méthanol, et avec un rendement de 59 %, le composé **9** de formule : 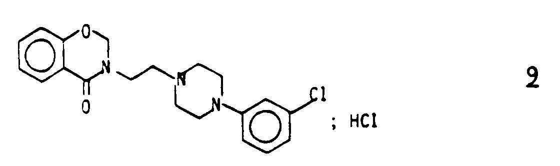
- formule brute : C₂₀H₂₃Cl₂N₃O₂
- masse moléculaire : 408,31
- cristaux blanc cassé
- point de fusion : 182°C
- IR (KBr) √CO amide : 1660 cm⁻¹
- RMN (DMSO d₆) δ : 2.8-3.4 (m,4H) ; 3.5-3.8 (m,6H) ; 4.11 (t,3H) ; 5.4 (s,2H) ; 6.6-7 (m,4H) ; 7.05-7.20 (m,2H) ; 7.3-7.5 (m,1H) ; 7.84 (dd,1H) ; 12.1 (s,1H) ; p.p.m.
- soluble dans le DMSO à 10 %.

### Exemple 10

### Chlorhydrate de dihydro-2,3 [(trifluorométhyl-3 phényl)-4 pipérazinyl)-2 éthyl]-3 4H-Benzoxazine-1,3 one-4

En partant de la trifluorométhyl-3 phénylpipérazine et de la dihydro-2,3 (méthanesulfonyloxy-2 éthyl)-3 4H-Benzoxazine-1,3 one-4, et en utilisant le procédé de l'exemple 1, on obtient, avec un rendement de 63 %, le composé **10** de formule : 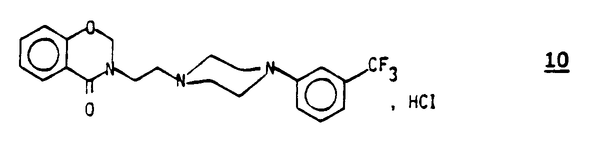
- formule brute : C₂₁H₂₃ClF₃N₃O₂
- masse moléculaire : 441,88
- cristaux pulvérulents blancs
- point de fusion : 196°C
- IR (KBr) √CO amide : 1670 cm⁻¹
- RMN (CDCl₃) δ : 2.9-3.2 (m,2H) ; 3.37 (t,2H) ; 3.5-3.9 (m,6H) ; 4.12 (t,2H) ; 5.39 (s,2H) ; 6.8-7.2 (m,5H) ; 7.25-7.5 (m,2H) ; 7.7 (dd,1H) ; 12.77 (s,1H) ; p.p.m.
- soluble dans le DMSO à 7 %.

### Exemple 11

### Chlorhydrate de dihydro-2,3 [(pyridine-2 yl)-4 pipérazinyl)-2 éthyl]-3 4H-Benzoxazine-1,3 one-4

L'utilisation du procédé de l'exemple 1 adapté à la (pyridine-2 yl pipérazine et à la dihydro-2,3 (méthanesulfonyloxy-2 éthyl)-3 4H-Benzoxazine-1,3 one-4, conduit, apàs recristallisation dans le méthanol, avec un rendement de 49 %, au composé **11** ayant pour formule : 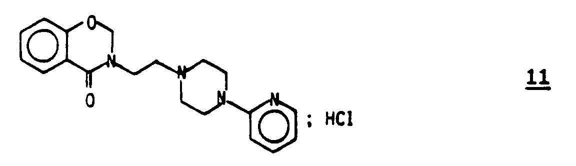
- formule brute : C₁₉H₂₃ClN₄O₂
- masse moléculaire : 374,86
- cristaux aciculaires blancs
- point de fusion : 196°C
- IR (KBr) √CO amide : 1661 cm⁻¹
- RMN (DMSO d₆) δ : 2.5-4.9 (m,13H) ; 5.42 (s,2H) ; 8.5-7.8 (m,2H) ; 6.9 (d,1H) ; 7.12 (t,1H) ; 7.4-7.7 (m,2H) ; 7.91 (dd,1H) ; 8.19 (d,1H) ; p.p.m.
- soluble dans l'eau à 0,3 %.

### Exemple 12

### Chlorhydrate de dihydro-2,3 [(pyrimidine-2 yl)-4 pipérazinyl)-2 éthyl]-3 4H-Benzoxazine-1,3 one-4

L'application du procédé décrit dans l'exemple 1 à la pyrimidyl-2 pipérazine condensée avec la dihydro-2,3 (méthanesulfonyloxy-2 éthyl)-3 4H-Benzoxazine-1,3 one-4, permet de préparer, avec un rendement de 39 % après purification dans un mélange isopropanol-méthanol, le composé **12** de formule : 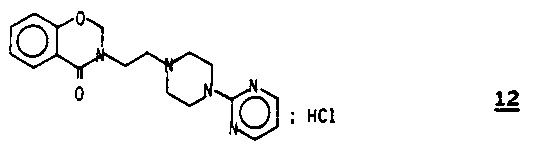
- formule brute : C₁₈H₂₂ClN₅O₂
- masse moléculaire : 375,86
- cristaux blancs
- point de fusion : 234°C
- IR (KBr) √CO amide : 1659 cm⁻¹
- RMN (DMSO d₆) δ : 2.9-3.53 (m,6H) ; 3.68 (m,2H) ; 3.92 (m,2H) ; 4.69 (m,2H) ; 5.45 (s,2H) ; 6.77 (t,1H) ; 7.0-7.25 (m,2H) ; 7.55 (t,1H) ; 7.81 (d,1H) ; 8.45 (d,2H) ; 11.15 (s,1H) ; p.p.m.
- soluble dans l'eau à 1,2 %.

### Exemple 13

### Chlorhydrate de dihydro-2,3 méthyl-6 [(méthoxy-2 phényl)-4 pipérazinyl)-2 éthyl]-3 4H-Benzoxazine-1,3 one-4

L'adaptation du procédé décrit dans l'exemple 1 à la (méthoxy-2 phényl) pipérazine condensée avec la méthyl-6 dihydro-2,3 (méthanesulfonyloxy-2 éthyl)-3 4H-Benzoxazine-1,3 one-4, donne, après recristallisation dans l'isopropanol, avec un rendement de 81 %, le composé **13** de formule : 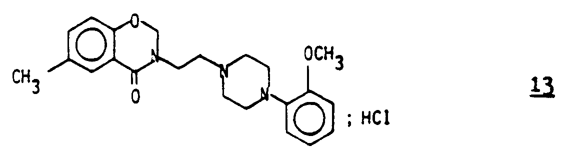
- formule brute : C₂₂H₂₈ClN₃O₃
- masse moléculaire : 417,937
- cristaux blancs
- point de fusion : 216°C
- IR (KBr) √CO amide : 1667 cm⁻¹
- RMN (CDCl₃) δ : 2.33 (s,1H) ; 3.1-3.44 (m,4H) ; 3.52 (d,4H) ; 3.72 (d,2H) ; 3.86 (s,3H) ; 4.19 (t,2H) ; 5.41 (s,2H) ; 6.7-7.15 (m,5H) ; 7.27 (d,1H) ; 7.70 (s,1H) ; 12.84 (s,1H) p.p.m.
- soluble dans le DMSO à 3 %.

### Exemple 14

### Chlorhydrate de chloro-6 dihydro-2,3 [((méthoxy-2 phényl)-4 pipérazinyl)-2 éthyl]-3 4H-Benzoxazine-1,3 one-4

La condensation de la chloro-6 dihydro-2,3 (méthanesulfonyloxy-2 éthyl)-3 4H-Benzoxazine-1,3 one-4, sur la (méthoxy-2 phényl) pipérazine selon le procédé de l'exemple 1, donne, après recristallisation dans l'éthanol, avec un rendement de 71 %, le composé **14** quant pour formule : 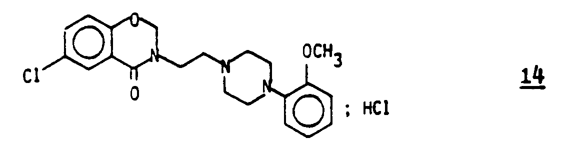
- formule brute : C₂₁H₂₅Cl₂N₃O₃
- masse moléculaire : 438,355
- cristaux blancs
- point de fusion : 228°C
- IR (KBr) √CO amide : 1671 cm⁻¹
- RMN (CDCl₃) δ : 3.1-3.42 (m,4H) ; 3.5 (d,4H) ; 3.72 (d,2H) ; 3.66 (s,3H) ; 4.20 (t,2H) ; 5.47 (s,2H) ; 6.8-7.2 (m,5H) ; 7.4 (dd,1H) ; 7.68 (d,1H) ; 12.83 (s,1H) ; p.p.m.
- soluble dans le DMSO à 1 %.

### Exemple 15

### Chlorhydrate de méthoxy-6 dihydro-2,3 [(méthoxy-2 phényl)-4 pipérazinyl)-2 éthyl]-3 4H-Benzoxazine-1,3 one-4

Par réaction de la dihydro-2,3 méthoxy-6 (méthanesulfonyloxy-2 éthyl)-3 4H-Benzoxazine-1,3 one-4, avec la (méthoxy-2 phényl) pipérazine selon le mode opératoire de l'exemple 1, et après purification dans l'isopropanol, on prépare, avec un rendement de 59 %, le composé **15** de formule : 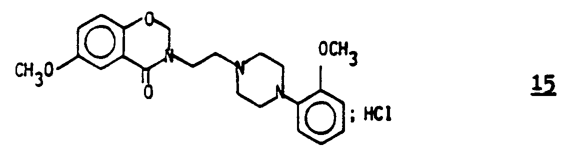
- formule brute : C₂₂H₂₈ClN₃O₄
- masse moléculaire : 433,936
- cristaux blancs
- point de fusion : 242°C
- IR (KBr) √CO amide : 1666 cm⁻¹
- RMN (CDCl₃) δ : 3.19 (m,2H) ; 3.35 (m,2H) ; 3.52 (m,4H) ; 3.6-3.9 (m,8H) ; 4.19 (t,2H) ; 5.4 (s,2H); 6.66-7.11 (m,6H) ; 7.36 (d,2H) ; 12.82 (s,1H); p.p.m.
- soluble dans le DMSO à 1,3 %.

### Exemple 16

### Chlorhydrate de dihydro-2,6 méthoxy-6 [(trifluorométhyl-3 phényl)-4 pipérazinyl)-2 éthyl]-3 4H-Benzoxazine-1,3 one-4

En adaptant le mode opératoire de l'exemple précédent à la (trifluorométhyl-3 phényl) pipérazine, on prépare, avec un rendement de 79 %, le composé **16** quant pour formule : 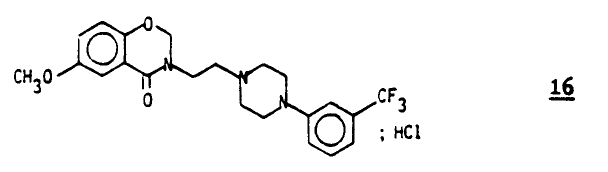
- formule brute : C₂₂H₂₅ClF₃N₃O₃
- masse moléculaire : 471,907
- cristaux blancs pulvérulents
- point de fusion : 198°C
- IR (KBr) √CO amide : 1671 cm⁻¹
- RMN (CDCl₃) δ : 3-3.3 (m,2H) ; 3.4-3.55 (m,2H) ; 3.6-4.05 (m,9H) ; 4.19 (t,2H) ; 6.92 (d,1H) ; 7.00-7.25 (m,4H) ; 7.3-7.5 (m,2H) ; 13.17 (s,1H) ; p.p.m.
- soluble dans le DMSO à 7 %.

### Exemple 17

### Chlorhydrate de dihydro-2,3 diméthoxy-6,7 [(méthoxy-2 phényl)-4 pipérazinyl)-4 butyl]-3 4H-Benzoxazine-1,3 one-4

Lorsque l'on condense la dihydro-2,3 diméthoxy-6,7 (méthanesulfonyloxy-4 butyl)-3 4H-Benzoxazine-1,3 one-4 sur la (méthoxy-2 phényl) pipérazine selon le procédé décrit dans l'exemple 1, on prépare, après recristallisation dans le méthanol bouillant, le composé **17** avec un rendement de 41 % et de formule : 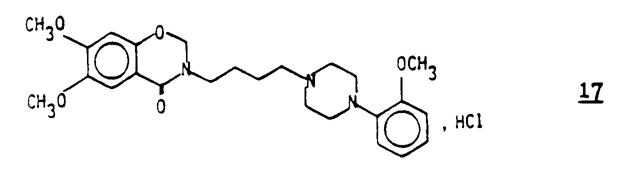
- formule brute : C₂₅H₃₄ClN₃O₅
- masse moléculaire : 492,016
- cristaux prismatiques blancs
- point de fusion : 240°C
- IR (KBr) √CO amide : 1650 cm⁻¹
- RMN (CDCl₃) δ : 1.6-1.9 (m,2H) ; 1.95-2.1 (m,2H) ; 2.9-3.3 (m,4H) ; 3.35-3.7 (m,8H) ; 3.8-4 (m,9H) ; 5.16 (s,2H) ; 8.46 (s,1H) ; 8.7-7.15 (m,4H) ; 7.32 (s,1H) ; 12.49 (s,1H) ; p.p.m.
- soluble dans l'eau à 0,2 %.

### Exemple 18

### Chlorhydrate de dihydro-2,3 diméthoxy-6,7 [((diméthoxy-3,4 phényl)-4 pipérazinyl)-4 butyl]-3 4H-Benzoxazine-1,3 one-4

L'utilisation du procédé décrit dans l'exemple précédent mais en utilisant la (diméthoxy-3,4 phényl) pipérazine comme matière première conduit, après purification dans un mélange méthanol-éthanol, au composé **18** avec un rendement de 83 % et ayant pour formule : 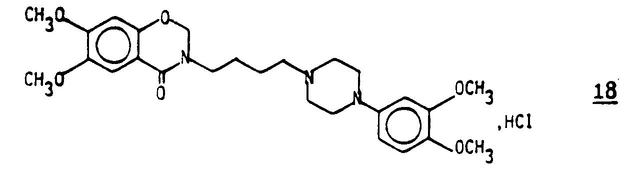
- formule brute : C₂₆H₃₆ClN₃O₆
- masse moléculaire : 522,042
- cristaux blancs
- point de fusion : 230°C
- IR (KBr) √CO amide : 1656 cm⁻¹
- RMN (CDCl₃) δ : 1.7-1.9 (m,2H) ; 1.9-2.2 (m,2H) ; 2.9-3.25 (m,4H) ; 3.3-3.8 (m,8H) ; 3.8-4.1 (m,12H) ; 5.17 (s,2H) ; 6.4-6.7 (m,3H) ; 6.77 (dd,1H) ; 7.31 (s,1H) ; 12.59 (s,1H) ; p.p.m.
- soluble dans le DMSO à 2 %.

### Exemple 19

### Hydrate du (dl) chlorhydrate de dihydro-2,3 méthyl-2 [((trifluorométhyl-3 phényl)-4 pipérazinyl)-2 éthyl]-3 4H-Benzoxazine-1,3 one-4

En utilisant le procédé décrit dans l'exemple 1 mais en partant de la dihydro-2,3 méthyl-2 (méthanesulfonyloxy-2 éthyl)-3 4H-Benzoxazine-1,3 one-4 et de la trifluorométhyl-3 phénylpipérazine comme matière première, on obtient, après purification dans l'alcool isopropylique avec un rendement de 74 % le composé **19** ayant pour formule : 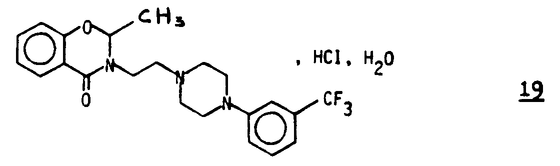
- formule brute : C₂₂H₂₇ClF₃N₃O₃
- masse moléculaire : 473 92
- cristaux blancs
- point de fusion : 161°C
- IR (KBr) √CO amide : 1670 cm⁻¹
- RMN (CDCl₃) δ : 1.63 (d,3H) ; 2.08 (s,2H : eau) ; 3.41 (m,2H) ; 3.66 (m,6H) ; 4.02 (m,1H) ; 4.24 (m,1H) ; 5.77 (q,1H) ; 6.95 (d,1H) ; 7.19 (m,4H) ; 7.44 (dd,1H) ; 7.67 (dd,1H) ; 13.12 (s,1H) ; p.p.m.
- soluble dans le DMSO à 2 %.

### EXPERIMENTATIONS BIOLOGIQUES

### 1) Etude pharmacologique: liaison 5HT₁A

Les études de liaison 5HT₁A ont été réalisées selon la technique de Peroutka (cf. Peroutka S.J., J. Neurochem. 47, p.529-40, 1986).
Des rats mâles Charles River sont assommés et décapités, le cerveau est prélevé et disséqué. Les différentes aires cérébrales obtenues sont utilisées ou conservées à moins 20°C. L'hippocampe est broyé au Polytron^{R} (20 secondes à 7) dans 20 volumes de tampon Tris-HCl 50 mM (pH 7,7 à 25°C) et centrifugé à 45000 g, 10 mn. Le culot est séparé, repris dans le même volume de tampon Tris et incube 10mn à 37°C puis recentrifugé 10 mn à 45000 g. Le culot est enfin repris dans 100 volumes de tampon Tris-HCl renfermant 10 µM de Pargyline, 4 mM CaCl₂ et 0,1 % d'acide ascorbique. Le mélange obtenu est homogénéisé au Dounce.

La liaison est réalisé à partir de 0,8 ml de la suspension membranaire précédente à laquelle on ajoute 0,1 ml de ligand ³H-8-OH-DPAT (à la concentration 1 nM) et soit 0,1 ml de tampon Tris (témoin), soit le composé de la précédente invention. Après incubation 30 mn à 25°C, le mélange est filtré sur GF/B (Whatman), rincé avec 5 ml de tampon Tris-HCl glacé. Le résidu et le filtre sont enfin introduits dans une fiole renfermant 3 ml de liquide scintillant Instagel (Packard) et la radioactivité est mesurée au compteur Packard (Tri-Carbs).

Les CI₅₀ sont déterminées graphiquement pour une concentration de ligand de 1 nM.

Le tableau I donne les CI₅₀ pour les récepteurs 5HT₁A pour certains dérivés de l'invention à titre d'exemple non limitatif.

| Composé relatif à l'exemple | CI₅₀ nM |
|---|---|
| 1 | 7 |
| 5 | 5 |
| 14 | 5 |
| 15 | 2,2 |

### 2) Applications thérapeutiques :

Compte tenu de leur activité pharmacologique, les dérivés de la présente invention peuvent être utilisés en thérapeutique humaine ou animale dans les troubles du système nerveux central ou cardiovasculaires.

Par suite de leur action sur le récepteur 5HT₁A, les composés de la présente invention sont plus précisément indiqués au niveau SNC dans le traitement des états dépressifs ou de l'anxiété, éventuellement dans les troubles du sommeil et la régulation de la prise de nourriture. D'autres composés sont plus précisément utiles au niveau cérébrovasculaire et cardiovasculaire dans le traitement de l'hypertension et éventuellement de la migraine.

Les composés de la présente invention sont aussi utilisés pour préparer des médicaments. Leur administration peut être réalisée par voie orale, parentérale ou rectale ; chaque dose est constituée d'un adjuvant pharmaceutique inerte facilitant la préparation du médicament et l'absorption du principe actif, pouvant être associé à un autre.

Ces médicaments peuvent se présenter sous forme de comprimés, gélules, suspensions, émulsions, sirops, suppositoires, solutions ou analogues.

L'administration du principe actif peut se faire à une dose journalière comprise entre 5 et 800 mg.

La préparation suivante est donnée à titre d'exemple non limitatif, les ingrédients ainsi que d'autres pouvant être introduits en d'autres proportions sans modifier la portée de l'invention.

### Exemple 20 : comprimés

| | |
|---|---|
| . Chlorhydrate de méthoxy-6 dihydro-2,3 [(méthoxy-2 phényl-4 pipérazinyl)-2 éthyl]-3 4H-Benzoxazine-1,3 one-4 | 25 mg |
| . Lactose | 15 mg |
| . Amidon de maïs | 50 mg |
| . Stéarate de magnésium | 5 mg |
| . Polyvinyl pyrrolidone | 10 mg |
| . Carboxyméthyl cellulose | 30 mg |

## Revendications

1. Dérivés de dihydro-2,3 (pipérazinyl alcoyl)-3 4H-Benzoxazine-1,3 ones-4 de formule I : 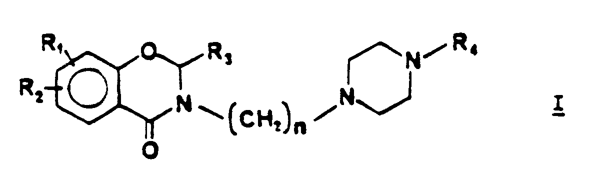 dans laquelle les substituants sont définis comme suit :
- R₁ et R₂ égaux ou différents représentent un hydrogène, un groupement alcoyle inférieur renfermant de 1 à 6 carbones, ramifié ou non ; un groupement alcoyloxy inférieur renfermant de 1 à 4 carbones ; un hydroxyle ; un nitro ; un amino substitué ou non par un groupement acyle ou un ou deux alcoyle renfermant chacun indépendamment 1 à 4C ; un groupement méthylsulfonylamino ; un halogène ;
- R₃ représente un hydrogène, un groupement aliphatique ramifié ou non, saturé ou non, renfermant de 1 à 5 C ;
- R₄ représente soit :
. un groupement phényle pouvant être substitué ou non par un ou plusieurs radicaux identiques ou différents choisis parmi les suivants :
- un groupement alcoyle inférieur renfermant de 1 à 6 C ; linéaire ou ramifié,
- un groupement alcoyloxy contenant de 1 à 6 C ; linéaire ou ramifié,
- un halogène,
- un trifluorométhyle,
- un groupement nitro,
- un groupement hydroxy,
- un amino substitué ou non par un ou plusieurs groupements alcoyle, un acyle ou un carboxylate renfermant tous trois de 1 à 4 C ;
- un groupement méthylsulfonylamino ;
. un groupement hétéroaryle monocyclique renfermant un ou deux atomes d'azote ;
- n peut prendre les valeurs de 2 à 6;
ainsi que les sels minéraux ou organiques de I thérapeutiquement acceptables éventuellement sous forme hydratée;
ainsi que les racémates et les différents énantiomères, ou leurs mélanges lorsque les composés de formule I renferment un carbone asymétrique.

2. Composés selon la revendication 1 caractérisé en ce que les radicaux R₁ et R₂ sont plus précisément choisis parmi les suivants : H, CH₃, CH₃CH₂, (CH₃)₂CH, CH₃O, CH₃CH₂O, Br, Cl, F, OH, NO₂, NH₂, CH₃CONH, CH₃SO₂NH, (CH₃)₂N.

3. Composés selon l'une des revendications 1 ou 2 caractérisés en ce que le radical R₃ est représenté par H, CH₃, CH₃CH₂, (CH₃)₂CH, CH₃-CH=CH.

4. Composés selon l'une des revendications 1 à 3 caractérisés en ce que le radical R₄ est représenté soit par :
- un phényle pouvant être ou non substitué par un ou plusieurs radicaux suivants : Br, Cl, F, CF₃, CH₃, CH₃CH₂, CH₃O, CH₃CH₂O, NO₂, OH, NH₂, CH₃CONH, CH₃SO₂NH, (CH₃)₂N,
- un groupement pyridyle,
- un groupement pyrimidinyle.

5. Composés de formule générale I selon les revendications 1 à 4 caractérisés par le fit qu'ils sont choisis parmi :
Chlorhydrate de dihydro-2,3 [((trifluorométhyl-3 phényl)-4 pipérazinyl)-4 butyl]-3 4H-Benzoxazine-1,3 one-4
Chlorhydrate de dihydro-2,3 [(phényl-4 pipérazinyl)-3 propyl]-3 4H-Benzoxazine-1,3 one-4
Dichlorhydrate de dihydro-2,3 [(phényl-4 pipérazinyl)-2 éthyl]-3 4H-Benzoxazine-1,3 one-4
Chlorhydrate de dihydro-2,3 [(phényl-4 pipérazinyl)-4 butyl]-3 4H-Benzoxazine-1,3 one-4
Dichlorhydrate de dihydro-2,3 [((méthoxy-2 phényl)-4 pipérazinyl)-2 éthyl]-3 4H-Benzoxazine-1,3 one-4
Chlorhydrate de dihydro-2,3 [((méthoxy-2 phényl)-4 pipérazinyl)-3 propyl]-3 4H-Benzoxazine-1,3 one-4
Hydrate du dichlorhydrate de dihydro-2,3 [((méthoxy-2 phényl)-4 pipérazinyl)-4 butyl]-3 4H-Benzoxazine-1,3 one-4
Chlorhydrate de dihydro-2,3 [((méthyl-2 phényl)-4 pipérazinyl)-2 éthyl]-3 4H-Benzoxazine-1,3 one-4
Chlorhydrate de dihydro-2,3 [((chloro-3 phényl)-4 pipérazinyl)-2 éthyl]-3 4H-Benzoxazine-1,3 one-4
Chlorhydrate de dihydro-2,3 [((trifluorométhyl-3 phényl)-4 pipérazinyl)-2 éthyl]-3 4H-Benzoxazine-1,3 one-4
Chlorhydrate de dihydro-2,3 [((pyridine-2 yl)-4 pipérazinyl)-2 éthyl]-3 4H-Benzoxazine-1,3 one-4
Chlorhydrate de dihydro-2,3 [((pyrimidine-2 yl)-4 pipérazinyl)-2 éthyl]-3 4H-Benzoxazine-1,3 one-4
Chlorhydrate de dihydro-2,3 méthyl-6 [((méthoxy-2 phényl)-4 pipérazinyl)-2 éthyl]-3 4H-Benzoxazine-1,3 one-4
Chlorhydrate de chloro-6 dihydro-2,3 [((méthoxy-2 phényl)-4 pipérazinyl)-2 éthyl]-3 4H-Benzoxazine-1,3 one-4
Chlorhydrate de méthoxy-6 dihydro-2,3 [((méthoxy-2 phényl)-4 pipérazinyl)-2 éthyl]-3 4H-Benzoxazine-1,3 one-4
Chlorhydrate de dihydro-2,3 méthoxy-6 [((trifluorométhyl-3 phényl)-4 pipérazinyl)-2 éthyl]-3 4H-Benzoxazine-1,3 one-4
Chlorhydrate de dihydro-2,3 diméthoxy-6,7 [((méthoxy-2 phényl)-4 pipérazinyl)-4 butyl]-3 4H-Benzoxazine-1,3 one-4
Chlorhydrate de dihydro-2,3 diméthoxy-6,7 [((diméthoxy-3,4 phényl)-4 pipérazinyl)-4 butyl]-3 4H-Benzoxazine-1,3 one-4
Hydrate du (dl) chlorhydrate de dihydro-2,3 méthyl-2 [((trifluorométhyl-3 phényl)-4 pipérazinyl)-2 éthyl]-3 4H-Benzoxazine-1,3 one-4.

6. Procédé de préparation des composés définis selon les revendications 1 à 4 caractérisé en ce que l'on condense un composé de formule II : 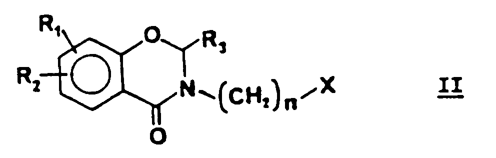 sur une pipérazine de formule **III** 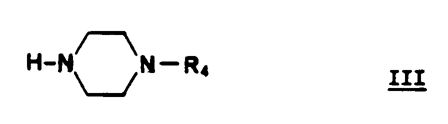 en présence ou non de base à une température comprise entre 20°C et 110°C où R₁, R₂, R₃, R₄ et n ont la même signification que précédemment et où X représente un halogène ou un radical arylsulfonate ou alcanesulfonate et préférentiellement un chlore, brome, tosylate et méthanesulfonate.

7. Procédé selon la revendication 6 caractérisé en ce que la base utilisée est la triéthylamine ou la pyridine.

8. Procédé selon la revendication 6 caractérisé en ce que la condensation est réalisée à une température comprise entre 50 et 100°C.

9. A titre de médicaments, utiles en particulier dans le traitement des troubles soit du système nerveux central et plus précisément comme antidépresseur, anxiolytique ou antimigraineux, soit aussi des troubles du système cardiovasculaire et plus précisément comme antihypertenseur, les composés définis selon les revendications 1 à 5.

10. Compositions pharmaceutiques caractérisées en ce qu'elles contiennent comme principe actif au moins un composé selon l'une des revendications 1 à 5 asssocié à un support pharmaceutique inerte.

## Claims

1. Derivatives of 2,3-dihydro-3-(piperazinylalkyl)-4H-1,3-benzoxazin-4-ones of formula **I** 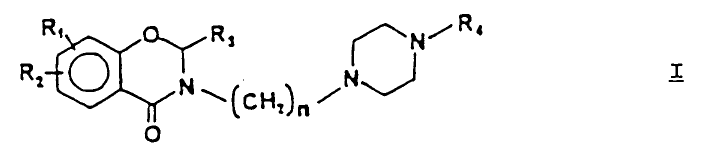 in which formula the substituents are defined as follows:
- R₁ and R₂, which are identical or different, represent a hydrogen, a lower alkyl group containing 1 to 6 carbons, which is branched or unbranched; a lower alkyloxy group containing 1 to 4 carbons; a hydroxyl; a nitro; an amino which is unsubstituted or substituted by an acyl group or one or two alkyls, each independently containing 1 to 4C; a methylsulphonylamino group; a halogen;
- R₃ represents a hydrogen, an aliphatic group which is branched or unbranched and saturated or unsaturated, containing 1 to 5C;
- R₄ represents either:
• a phenyl group which can be unsubstituted or substituted by one or more identical or different radicals, chosen from amongst the following:
- a lower alkyl group containing 1 to 6C, which is linear or branched,
- an alkyloxy group containing 1 to 6C, which is linear or branched,
- a halogen,
- a trifluoromethyl,
- a nitro group,
- a hydroxy group,
- an amino which is unsubstituted or substituted by one or more alkyl groups, an acyl or a carboxylate, all three containing 1 to 4C;
- a methylsulphonylamino group,
• a monocyclic heteroaryl group containing one or two nitrogen atoms;
- n can assume the values 2 to 6;
as well as the therapeutically acceptable inorganic or organic salts of **I**, optionally in hydrated form; and also the racemates and the various enantiomers, or their mixtures when the compounds of the formula **I** contain an asymmetric carbon.

2. Compounds according to Claim 1, characterized in that the radicals R₁ and R₂ are more precisely chosen from amongst the following: H, CH₃, CH₃CH₂, (CH₃)₂CH, CH₃O, CH₃CH₂O, Br, Cl, F, OH, NO₂, NH₂, CH₃CONH, CH₃SO₂NH, (CH₃)₂N.

3. Compounds according to one of Claims 1 or 2, characterized in that the radical R₃ is represented by: H, CH₃, CH₃CH₂, (CH₃)₂CH, CH₃-CH=CH.

4. Compounds according to one of Claims 1 to 3, characterized in that the radical R₄ is represented either by:
- a phenyl which can be unsubstituted or substituted by one or more of the following radicals: Br, Cl, F, CF₃, CH₃, CH₃CH₂, CH₃O, CH₃CH₂O, NO₂, OH, NH₂, CH₃CONH, CH₃SO₂NH, (CH₃)₂N,
- a pyridyl group,
- a pyrimidinyl group.

5. Compounds of general formula **I** according to Claims 1 to 4, characterized in that they are chosen from amongst:
Hydrochloride of 2,3-dihydro-3-[4-(4-(3-trifluoromethylphenyl)piperazinyl)butyl]-4H-1,3-benzoxazin-4-one
Hydrochloride of 2,3-dihydro-3-[3-(4-phenylpiperazinyl)propyl]-4H-1,3-benzoxazin-4-one
Dihydrochloride of 2,3-dihydro-3-[2-(4-phenylpiperazinyl)ethyl]-4H-1,3-benzoxazin-4-one
Hydrochloride of 2,3-dihydro-3-[4-(4-phenylpiperazinyl)butyl]-4H-1,3-benzoxazin-4-one
Dihydrochloride of 2,3-dihydro-3-[2-(4-(2-methoxyphenyl)piperazinyl)ethyl]-4H-1,3-benzoxazin-4-one
Hydrochloride of 2,3-dihydro-3-[3-(4-(2-methoxyphenyl)piperazinyl)propyl]-4H-1,3-benzoxazin-4-one
Hydrate of the dihydrochloride of 2,3-dihydro-3-[4-(4-(2-methoxyphenyl)piperazinyl)butyl]-4H-1,3-benzoxazin-4-one
Hydrochloride of 2,3-dihydro-3-[2-(4-(2-methylphenyl)piperazinyl)ethyl]-4H-1,3-benzoxazin-4-one
Hydrochloride of 2,3-dihydro-3-[2-(4-(3-chlorophenyl)piperazinyl)ethyl]-4H-1,3-benzoxazin-4-one
Hydrochloride of 2,3-dihydro-3-[2-(4-(3-trifluoromethylphenyl)piperazinyl)ethyl]-4H-1,3-benzoxazin-4-one
Hydrochloride of 2,3-dihydro-3-[2-(4-(2-pyridinyl)piperazinyl)ethyl]-4H-1,3-benzoxazin-4-one
Hydrochloride of 2,3-dihydro-3-[2-(4-(2-pyrimidinyl)piperazinyl)ethyl]-4H-1,3-benzoxazin-4-one
Hydrochloride of 2,3-dihydro-6-methyl-3-[2-(4-(2-methoxyphenyl)piperazinyl)ethyl]-4H-1,3-benzoxazin-4-one
Hydrochloride of 6-chloro-2,3-dihydro-3-[2-(4-(2-methoxyphenyl)piperazinyl)ethyl]-4H-1,3-benzoxazin-4-one
Hydrochloride of 6-methoxy-2,3-dihydro-3-[2-(4-(2-methoxyphenyl)piperazinyl)ethyl]-4H-1,3-benzoxazin-4-one
Hydrochloride of 2,3-dihydro-6-methoxy-3-[2-(4 (3-trifluoromethylphenyl)piperazinyl)ethyl]-4H-1,3-benzoxazin-4-one
Hydrochloride of 2,3-dihydro-6,7-dimethoxy-3-[4-(4-(2-methoxyphenyl)piperazinyl)butyl]-4H-1,3-benzoxazin-4-one
Hydrochloride of 2,3-dihydro-6,7-dimethoxy-3-[4-(4-(3,4-dimethoxyphenyl)piperazinyl)butyl]-4H-1,3-benzoxazin-4-one
Hydrate of the (dl) hydrochloride of 2,3-dihydro-2-methyl-3-[2-(4-(3-trifluoromethylphenyl)piperazinyl)ethyl]-4H-1,3-benzoxazin-4-one

6. Process of preparation of the compounds defined according to Claims 1 to 4, characterized in that a compound of formula **II**: 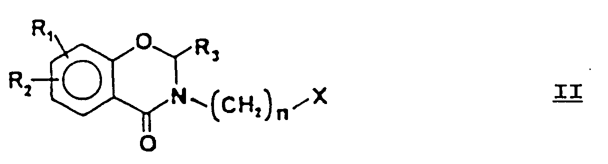 is condensed onto a piperazine of the formula **III** 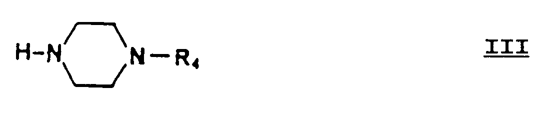 in the presence or absence of a base at a temperature between 20°C and 110°C, where R₁, R₂, R₃, R₄ and n have the same meaning as above and where X represents a halogen or an arylsulphonate or alkanesulphonate radical and preferentially a chlorine, bromine, tosylate or methanesulfonate.

7. Process according to Claim 6, characterized in that the base used is triethylamine or pyridine.

8. Process according to Claim 6, characterized in that the condensation is carried out at a temperature between 50 and 100°C.

9. The compounds defined according to Claims 1 to 5, as medicaments, useful in particular in the treatment of disorders either of the central nervous system and more precisely as an antidepressant, an anxiolytic or an antimigraine agent, or else of disorders of the cardiovascular system and more precisely as an antihypertensive.

10. Pharmaceutical compositions, characterized in that they contain as active principle at least one compound according to one of Claims 1 to 5 combined with an inert pharmaceutical support.

## Patentansprüche

1. Derivate von 2,3-Dihydro-3-(piperazinylalkyl)-1,3-4H-benzoxazin-4-onen der Formel (I) 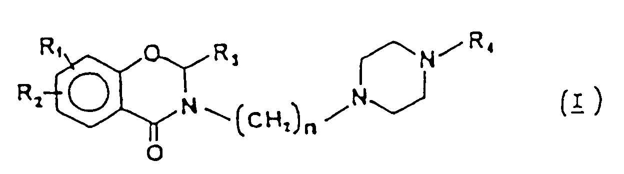 in der die Substituenten die folgenden Bedeutungen haben:
R₁ und R₂ die gleich oder verschieden sind, stehen für ein Wasserstoffatom, eine verzweigte oder unverzeigte niedere Alkylgruppe mit 1 bis 6 Kohlenstoffatomen; eine niedere Alkyloxygruppe mit 1 bis 4 Kohlenstoffatomen; eine Hydroxylgruppe; eine Nitrogruppe; eine Aminogruppe, die unsubstituiert oder substituiert ist durch eine Acylgruppe oder eine oder zwei Alkylgruppen, die jeweils unabhängig voneinander 1 bis 4 Kohlenstoffatome enthalten; eine Methylsulfonylaminogruppe; ein Halogenatom;
R₃ steht für ein Wasserstoffatom, eine gesättigte oder ungesättigte, verzweigte oder unverzeigte aliphatische Gruppe mit 1 bis 5 Kohlenstoffatomen;
R₄ steht für
. entweder eine Phenylgruppe, die unsubstituiert oder substituiert sein kann durch einen oder mehrere identische oder verschiedene Reste, ausgewählt aus den folgenden:
- eine lineare oder verzweigte niedere Alkylgruppe mit 1 bis 6 Kohlenstoffatomen;
- eine lineare oder verzweigte Alkylgruppe mit 1 bis 6 Kohlenstoffatomen;
- ein Halogenatom,
- eine Trifluoromethylgruppe,
- eine Nitrogruppe,
- eine Hydroxygruppe,
- eine Aminogruppe, die unsubstituiert oder substituiert ist durch eine oder mehrere Alkylgruppen, eine Acylgruppe oder eine Carboxylatgruppe, die alle drei 1 bis 4 Kohlenstoffatome enthalten;
- eine Methylsulfonylaminogruppe;
. oder eine monocyclische Heteroarylgruppe, die ein oder zwei Stickstoffatome enthält; und
n kann die Werte 2 bis 6 haben;
sowie die therapeutisch akzeptablen mineralischen oder organischen Salze von (I), gegebenenfalls in der hydratisierten Form;
sowie die Racemate und die verschiedenen Enantiomeren oder ihre Mischungen, wenn die Verbindungen der Formel (I) ein asymmetrisches Kohlenstoffatom enthalten.

2. Verbindungen nach Anspruch 1, dadurch gekennzeichnet, daß die Reste R₁ und R₂ insbesondere ausgewählt werden aus den folgenden:
H, CH₃, CH₃CH₂, (CH₃)₂CH, CH₃O, CH₃CH₂O, Br, Cl, F, OH, NO₂, NH₂, CH₃CONH, CH₃SO₂NH, (CH₃)₂N.

3. Verbindungen nach einem der Ansprüche 1 oder 2, dadurch gekennzeichnet, daß die Reste R₃ stehen für H, CH₃, CH₃CH₂, (CH₃)₂CH, CH₃CH=CH.

4. Verbindungen nach einem der Ansprüche 1 bis 3, dadurch gekennzeichnet, daß der Rest R₄ steht für:
- eine Phenylgruppe, die unsubstituiert oder substituiert sein kann durch einen oder mehrere der folgenden Reste: Br, Cl, F, CF₃, CH₃, CH₃CH₂, CH₃O, CH₃CH₂O, NO₂, OH, NH₂, CH₃CONH, CH₃SO₂NH, (CH₃)₂N,
- eine Pyridylgruppe,
- eine Pyrimidinylgruppe.

5. Verbindungen der allgemeinen Formel (I) nach den Ansprüchen 1 bis 4, dadurch gekennzeichnet, daß sie ausgewählt werden aus der Gruppe:
Chlorhydrat von 2,3-Dihydro-3-[4-(4-(3-trifluoromethylphenyl)-piperazinyl)-butyl]-1,3-4H-benzoxazin-4-on;
Chlorhydrat von 2,3-Dihydro-3-[3-(4-phenyl-piperazinyl)propyl]-1,3-4H-benzoxazin-4-on;
Dichlorhydrat von 2,3-Dihydro-3-[2-(4-phenyl-piperazinyl)ethyl]-1,3-4H-benzoxazin-4-on;
Chlorhydrat von 2,3-Dihydro-3-[4-(4-phenyl-piperazinyl)butyl]-1,3-4H-benzoxazin-4-on;
Dichlorhydrat von 2,3-Dihydro-3-[2-(4-(2-methoxy-phenyl)piperazinyl)-ethyl]-1,3-4H-benzoxazin-4-on;
Chlorhydrat von 2,3-Dihydro-3-[3-(4-(2-methoxy-phenyl)-piperazinyl)-propyl]-1,3-4H-benzoxazin-4-on;
Hydrat des Dichlorhydrats von 2,3-Dihydro-3-[4-(4-(2-methoxy-phenyl)-piperazinyl)-butyl]-1,3-4H-benzoxazin-4-on;
Chlorhydrat von 2,3-Dihydro-3-[2-(4-(2-methyl-phenyl)-piperazinyl)-ethyl]-1,3-4H-benzoxazin-4-on;
Chlorhydrat von 2,3-Dihydro-3-[2-(4-(3-chloro-phenyl)-piperazinyl)-ethyl]-1,3-4H-benzoxazin-4-on;
Chlorhydrat von 2,3-Dihydro-3-[2-(4-(3-trifluoromethylphenyl)-piperazinyl)-ethyl]-1,3-4H-benzoxazin-4-on;
Chlorhydrat von 2,3-Dihydro-3-[2-(4-(2-pyridin-yl)-piperazinyl)-ethyl]-1,3-4H-benzoxazin-4-on;
Chlorhydrat von 2,3-Dihydro-3-[2-(4-(2-pyrimidin-yl)-piperazinyl)-ethyl]-1,3-4H-benzoxazin-4-on;
Chlorhydrat von 2,3-Dihydro-6-methyl-3-[2-(4-(2-methoxyphenyl)-piperazinyl)-ethyl]-1,3-4H-benzoxazin-4-on;
Chlorhydrat von 6-Chloro-2,3-dihydro-3-[2-(4-(2-methoxyphenyl)-piperazinyl)-ethyl]-1,3-4H-benzoxazin-4-on;
Chlorhydrat von 6-Methoxy-2,3-dihydro-3-[2-(4-(2-methoxyphenyl)-piperazinyl)-ethyl]-1,3-4H-benzoxazin-4-on;
Chlorhydrat von 2,3-Dihydro-6-methoxy-3-[2-(4-(3-trifluoromethyl-phenyl)-piperazinyl)-ethyl]-1,3-4H-benzoxazin-4-on;
Chlorhydrat von 2,3-Dihydro-6,7-dimethoxy-3-[4-(4-(2-methoxy-phenyl)-piperazinyl)-butyl]-1,3-4H-benzoxazin-4-on;
Chlorhydrat von 2,3-Dihydro-6,7-dimethoxy-3-[4-(4-(3,4-dimethoxy-phenyl)-piperazinyl)-butyl]-1,3-4H-benzoxazin-4-on;
Hydrat des (dl)Chlorhydrats von 2,3-Dihydro-2-methyl-3-[2-(4-(4-(3-trifluoromethyl-phenyl)-piperazinyl)-ethyl]-1,3-4H-benzoxazin-4-on.

6. Verfahren zur Herstellung der Verbindungen nach den Ansprüchen 1 bis 4, dadurch gekennzeichnet, daß man eine Verbindung der Formel (II) 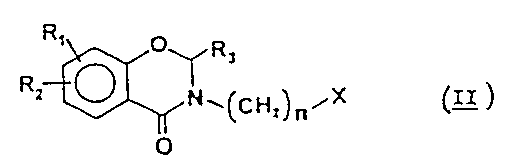 an ein Piperazin der Formel (III) 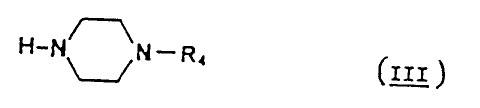 in Gegenwart oder Abwesenheit einer Base bei einer Temperatur zwischen 20 und 110°C kondensiert,
worin R₁, R₂, R₃, R₄ und n die gleichen Bedeutungen wie oben haben und X für ein Halogenatom oder einen Arylsulfonat- oder Alkansulfonat-Rest und vorzugsweise für Chlor, Brom, Tosylat und Methansulfonat steht.

7. Verfahren nach Anspruch 6, dadurch gekennzeichnet, daß als Base Triethylamin oder Pyridin verwendet wird.

8. Verfahren nach Anspruch 6, dadurch gekennzeichnet, daß die Kondensation bei einer Temperatur zwischen 50 und 100°C durchgeführt führt.

9. Verbindungen nach den Ansprüchen 1 bis 5 als Arzneimittel, die insbesondere bei der Behandlung von Störungen des Zentralnervensystems und speziell als Antidepressiva, Anxiolytika oder Antimigränemittel, oder auch bei Störungen des Cardiovasculärsystems und insbesondere als Antihypertensiva verwendbar sind.

10. Pharmazeutische Zusammensetzungen, dadurch gekennzeichnet, daß sie als aktives Prinzip (Wirkstoff) mindestens eine Verbindung nach einem der Ansprüche 1 bis 5 in Kombination mit einem inerten pharmazeutischen Träger enthalten.
